# EUROPÄISCHE PATENTSCHRIFT

(11) **EP 0 761 158 B1**
(45) Veröffentlichungstag und Bekanntmachung des Hinweises auf die Patenterteilung: **15.03.2000**
(21) Anmeldenummer: 96113547.2
(22) Anmeldetag: 23.08.1996
(51) Int. Cl.: A61B 1/00, A61B 19/00, A61B 19/08, A61C 1/16

(54) **Folienschlauchabdeckung**
Tubular foil cover
Recouvrement en forme de film tubulaire

(30) Priorität: 25.08.1995 DE 29513692 U
(43) Veröffentlichungstag der Anmeldung: 12.03.1997
(73) Patentinhaber: Udo Heisig GmbH, 85609 Aschheim-Dornach (DE)
(72) Erfinder: Heisig, Udo, 85521 Hohenbrunn-Riemerling (DE)
(74) Vertreter: Feldkamp, Rainer, Dipl.-Ing.

(56) Entgegenhaltungen:
- EP-A- 0 371 909
- DE-U- 9 304 063
- DE-U- 9 418 340
- US-A- 5 274 500

## Beschreibung

Die Erfindung bezieht sich auf eine Folienschlauchabdeckung für medizinische Geräte der im Oberbegriff des Anspruchs 1 genannten Art.

Bei vielen medizinischen Geräten, beispielsweise bei Endoskopen oder Arthroskopiekameras sind diese medizinischen Geräte mit Steuer- und Auswerteeinrichtungen über langgestreckte Schläuche und/oder Kabel verbunden, die mit sterilen Abdeckungen versehen sein müssen.

Zu diesem Zweck ist es bereits bekannt, Folienschlauchabdeckungen mit Folienschläuchen zu verwenden, die vor dem Gebrauch mit Hilfe einer sogenannten Steckfaltung auf eine sehr kurze Länge teleskopartig zusammengeschoben oder gefaltet sind, so daß ihre Handhabung beim Aufschieben auf die langgestreckten Schläuche und/oder Kabel erleichtert wird. Dieser Folienschlauch weist an seinem ersten Ende Hilfseinrichtungen zum Öffnen des Querschnittes des Folienschlauchs zum Erleichtern des Einsetzens von Teilen des medizinischen Gerätes auf, wobei diese Hilfseinrichtungen beispielsweise durch auf der Innenseite des Folienschlauches angeordnete Laschen gebildet sein können, die über dieses erste Ende des Folienschlauches hinaus vorspringen.

Weiterhin ist es bekannt, in dieses offene Ende zwei aufeinanderliegende Kartonblätter einzulegen, wobei durch Drücken auf die Längskanten dieser Kartonblätter ein Aufspreizen allgemein entsprechend der üblichen Knickverschlüsse von Taschen und dergleichen erzielt wird. Hierbei besteht jedoch die Gefahr, daß sich die beiden Kartonblätter bei Druck auf ihre Längskanten nicht voneinander trennen sondern zusammen in der einen oder anderen Richtung ausbeulen, so daß sich keine Öffnung des Innenquerschnittes des Folienschlauches ergibt.

Als Stand der Technik für die Erfindung wird auf die EP-A-371 909 verwiesen. Der Erfindung liegt die Aufgabe zugrunde, eine Folienschlauchabdeckung der eingangs genannten Art zu schaffen, die ein bequemes und zuverlässiges Öffnen des Querschnittes des Folienschlauches zum Erleichtern des Einsetzens von Teilen des medizinischen Gerätes ermöglicht.

Diese Aufgabe wird durch die im Anspruch 1 angegebenen Merkmale gelöst.

Vorteilhafte Ausgestaltungen und Weiterbildungen der Erfindung ergeben sich aus den Unteransprüchen.

Durch die erfindungsgemäße Ausgestaltung der Folienschlauchabdeckung ergibt sich ein einfaches Öffnen des Querschnittes des zur Aufbewahrung und zum Transport flachgelegten Folienschlauches, da die rohrförmige Manschette aufgrund ihrer längsverlaufenden Sollknickstellen bei einem Druck auf die ersten diametral gegenüberliegenden bzw. im flachgelegten Zustand des Folienschlauches längs dessen Längskanten verlaufenden Sollknickstellen eine Öffnung der Manschette in ihren rohrförmigen, beispielsweise viereckigen oder rechteckigen Zustand erreicht wird.

Durch die aus dem Folienschlauch heraus vorspringenden Laschen, die in Richtung auf eine zur Längsachse der Manschette senkrechte Richtung nach außen aufklappbar sind, läßt sich eine sichere Trennung zwischen sterilen und nicht sterilen Personen und Teilen erreichen, da beispielsweise die Laschen über eine das erste Ende des Folienschlauches greifende Hand aufgeklappt werden und diese Hand abdecken.

Die Manschette ist weiterhin vorzugsweise mit mindestens einer weiteren aus dem Folienschlauch heraus vorspringenden Lasche versehen, die sich erweiternde Schlitze zur Aufnahme von Kabeln, Schläuchen und dergleichen aufweist, die an dieser Lasche sicher festgelegt werden können.

Die Manschette kann beispielsweise auf steiferem Kunststoffmaterial oder auch aus Kartonmaterial hergestellt sein. Insbesondere dann, wenn die Manschette aus Kartonmaterial hergestellt ist, ist es aus Gründen der Abfalltrennung vorzugsweise vorgesehen, daß diese Manschette über lösbare Verbindungseinrichtungen, beispielsweise lösbare Klebe- oder Schweißnähte mit dem Folienschlauch verbunden ist, so daß die Manschette nach Gebrauch von dem Folienschlauch trennbar ist, damit diese Teile getrennt entsorgt werden können.

Ein Ausführungsbeispiel der Erfindung wird im folgenden anhand der Zeichnungen noch näher erläutert.

In der Zeichnung zeigen:
Fig. 1 eine in Längsrichtung verkürzte Darstellung einer Ausführung der Folienschlauchabdeckung,
Fig. 2 eine Teilansicht der Manschette der Folienschlauchabdeckung nach Fig. 1,
Fig. 3 eine vergrößerte Ansicht der in Fig. 2 durch die strichpunktierte Umrandung II dargestellten Einzelheit.

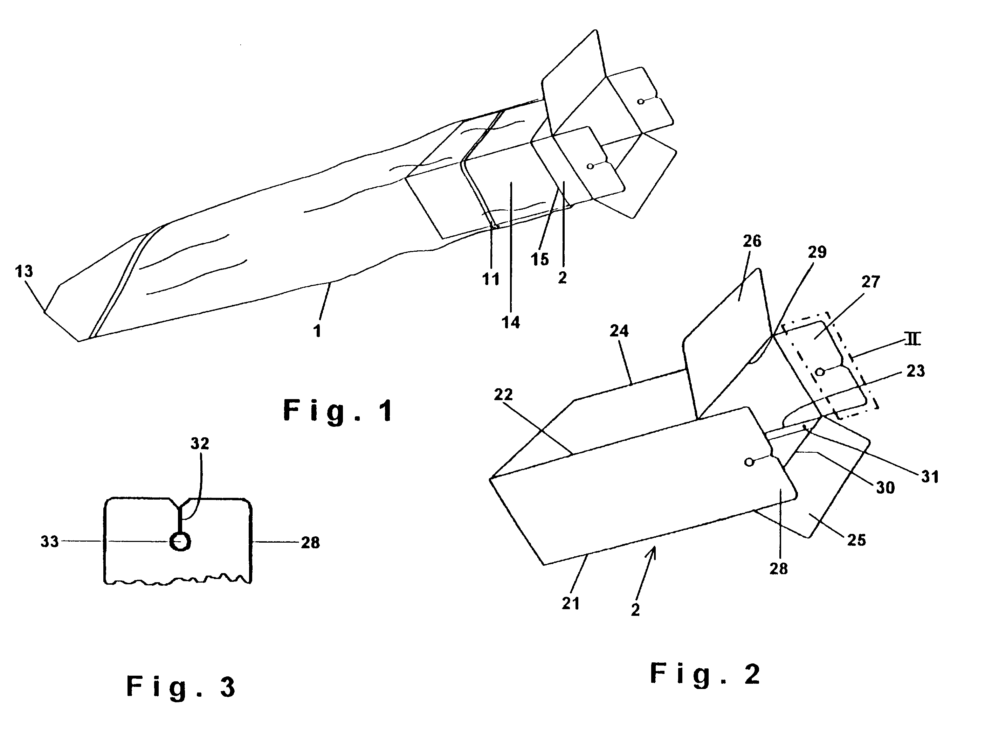

Die in Fig. 1 dargestellte Folienschlauchabdeckung besteht aus einem langgestreckten Folienschlauch, der durch eine Steckfaltung beispielsweise mit Faltungen an der mit 11 bezeichneten Stelle teleskopartig in seiner Länge verkürzt ist und ein erstes Ende 14 aufweist, in das eine Manschette 2 konzentrisch eingesteckt ist. Das andere Ende 13 ist beispielsweise durch Schweißnäht in seinem Querschnitt verringert und dient beispielsweise zur Aufnahme der Optik einer Endoskopkamera. An diesem Ende 13 kann ein nicht dargestellter Klebestreifen angeordnet sein, der zur sicheren Verbindung mit der Optik der Endoskopkamera dient.

Obwohl die Manschette bei der in Fig. 1 dargestellten Ausführungsform in das Innere des ersten Endes 14 des Folienschlauches 1 eingesteckt ist, ist es selbstverständlich genauso möglich, diesen Folienschlauch in das Innere der Manschette einzuschieben.

In jedem Fall ist jedoch die Manschette vorzugsweise durch eine Klebe- oder Schweißnaht 15 mit dem ersten Ende 14 des Folienschlauches 1 lösbar verbunden, was insbesondere dann von Bedeutung ist, wenn der Folienschlauch 1 und die Manschette 2 aus unterschiedlichen Materialien bestehen, die getrennt entsorgt werden müssen. In diesem Fall ist eine lösbare Ausgestaltung der Verbindung zwischen dem Folienschlauch 1 und der Manschette 2 zweckmäßig, um diese Trennung leicht durchführen zu können.

Bei der dargestellten Ausführungsform läßt sich die Manschette 2 in eine langgestreckt rechteckförmige Gestalt aufspreizen, und zu diesem Zweck ist die Manschette 2 mit einer Anzahl von in ihrer Axialrichtung längsverlaufenden Sollknickstellen 21, 22, 23, 24 versehen, von denen zwei diametral gegenüberliegende erste Sollknicklinien 21, 24 im flachgelegten Zustand des Folienschlauches entlang dessen Seitenkanten zu liegen kommen. Zwischen diesen beiden ersten Sollknicklinien 21, 24 sind weitere Sollknicklinien 22, 23 angeordnet, die bei Druckausübung auf die ersten beiden Sollknicklinien 21, 24 eine Öffnung des Querschnittes der rohrförmigen Manschette bewirken.

Die rohrförmige Manschette 2 kann beispielsweise aus Kartonmaterial hergestellt sein, wobei die beiden Enden eines Kartonstreifens durch eine bei 31 gezeigte schmale Lasche miteinander verbunden, beispielsweise verklebt sind. Die Sollknicklinien 21 bis 24 sind beispielsweise durch Einprägungen gebildet, die derart ausgestaltet sind, daß die rohrförmige Manschette 2 selbst im flachgelegten Zustand eine Tendenz hat, sich aufzuspreizen.

Obwohl in den Fig. 1 und 2 eine rohrförmige Manschette mit einem langgestreckt rechteckigen Querschnitt gezeigt ist, kann diese Manschette nach dem Öffnen genauso einen quadratischen Querschnitt oder einen vieleckigen Querschnitt durch entsprechende Wahl und Anordnung weiterer Sollknicklinien zwischen den ersten beiden Sollknicklinien 21, 24 aufweisen.

Im Hinblick auf eine sichere Handhabung kann die Manschette 2 weiterhin zwei sich aus dem Folienschlauch 1 heraus erstreckende Laschen 25, 26 aufweisen, die über Querknicklinien 29, 30 mit dem Hauptteil der Manschette 2 verbunden und in Richtung auf eine zur Längsachse der Manschette 2 senkrechte Ricihtung nach außen hin abklappbar sind. Beim Einsetzen beispielsweise einer Kamera durch die rohrförmige Manschette hindurch in den Folienschlauch kann dieses erste Ende des Folienschlauches bzw. die Manschette 2 von einer ersten Person mit einer Hand festgehalten werden, wobei sich ein automatisches Aufspreizen der rohrförmigen Manschette ergibt, und eine zweite Person kann die Kamera in das Innere der rohrförmigen Manschette einführen, ohne daß die Gefahr einer Berührung zwischen den Händen der beiden Personen besteht, da die die Folienschlauchabdeckung haltende Hand durch die Laschen 25, 26 abgedeckt ist.

Gemäß einer weiteren vorteilhaften Ausgestaltung der Folienschlauchabdeckung weist die Manschette 2 weiterhin zumindestens eine weitere aus dem Folienschlauch heraus vorspringende Lasche 27, 28 auf, die gemäß Fig. 3 sich erweiternde Schlitze 32, 33 zur Aufnahme von Kabeln, Schläuchen oder dergleichen aufweist, die in diesen Schlitzen sicher festgelegt werden können.

Wenn mehr als eine Lasche 27, 28 vorgesehen ist, so sind die sich erweiternden Schlitze 32, 33 dieser beiden Laschen vorzugsweise mit unterschiedlich großen Erweiterungen 33 versehen, um eine Anpassung an unterschiedlich Stärken der Schläuche oder Kabel zu erzielen.

## Patentansprüche

1. Folienschlauchabdeckung für medizinische Geräte, mit einem langgestreckten Folienschlauch, der zur Aufbewahrung und zum Transport flachgelegt und durch teleskopartige Steckfaltung in seiner Länge verkürzt und bei Gebrauch auf die volle Länge ausziehbar ist, und der ein erstes und ein zweites Ende aufweist, von denen zumindestens das erste Ende Hilfseinrichtungen zum Öffnen des Querschnittes des Folienschlauchs zum Erleichtern des Einsetzens von Teilen des medizinischen Gerätes aufweist,
wobei die Hilfseinrichtungen, die in einem aufgefalteten Zustand eine rohrförmige Manschette (2) bilden kann, eng anliegend konzentrisch in das erste Ende (14) des Folienschlauches (1) eingeschoben ist und über dieses erste Ende (14) nach außen hin vorspringt, und wobei die Manschette (2) aus steiferem Material als der Folienschlauch (1) besteht, dadurch gekennzeichnet, daß die Manschette (2) in ihrer Axialrichtung mit mindestens vier längsverlaufenden Sollknicklinien (21, 22, 23, 24) versehen ist, von denen zwei erste diametral gegenüberliegende Sollknicklinien (21, 24) eine flache Faltung der Manschette (2) und die zwischen diesen beiden ersten Sollknicklinien (21, 24) liegenden weiteren Sollknicklinien (22, 23) ein rohrförmiges Auffalten der Manschette (2) und damit des ersten Endes des Folienschlauches (1) durch Druck auf die ersten Sollknicklinien (21, 24) ermöglichen.

2. Folienschlauchabdeckung nach Anspruch 1,
dadurch gekennzeichnet, daß die Manschette (2) zwei aus dem Folienschlauch (1) heraus vorspringende Laschen (25, 26) aufweist, die über Querknicklinien (29, 30) mit dem Hauptteil der Manschette (2) verbunden und in Richtung auf eine zur Längsachse der Manschette (2) senkrechte Richtung nach außen hin abklappbar sind.

3. Folienschlauchabdeckung nach Anspruch 1 oder 2,
dadurch gekennzeichnet, daß die Manschette (2) mindestens eine weitere aus dem Folienschlauch heraus vorspringende Lasche (27, 28) aufweist, die sich erweiternde Schlitze (32, 33) zur Aufnahme von Kabeln, Schläuchen und dergleichen aufweist.

4. Folienschlauchabdeckung nach einem der vorhergehenden Ansprüche,
dadurch gekennzeichnet, daß die Manschette (2) aus Kartonmaterial hergestellt ist.

5. Folienschlauchabdeckung nach einem der vorhergehenden Ansprüche,
dadurch gekennzeichnet, daß das erste Ende (14) des Folienschlauches (1) an der rohrförmigen Manschette (2) durch von Hand lösbare Befestigungsmittel (15) verbunden ist.

6. Folienschlauchabdeckung nach Anspruch 5,
dadurch gekennzeichnet, daß die Befestigungsmittel durch eine lösbare Schweißverbindung (15) gebildet sind.

7. Folienschlauchabdeckung nach einem der vorhergehenden Ansprüche,
dadurch gekennzeichnet, daß in der Umfangsrichtung der rohrförmigen Manschette (2) die zwischen den beiden ersten Sollknicklinien (21, 24) liegende(n) Sollknicklinien(n) (22, 23) unterschiedliche Abstände von den jeweils benachbarten Sollknicklinien derart aufweisen, daß sich beim Öffnen des Querschnittes der rohrförmigen Manschette (2) bzw. des Folienschlauchs (1) ein langgestreckter rechteckiger Querschnitt ergibt.

## Claims

1. Tubular film covering for medical apparatus, comprising an elongate tubular film, which is laid flat and reduced in length by telescopic tucked folding for storage and transportation and is extended to its full length when used, and which comprises a first and a second end, of which at least the first end comprises auxiliary means for opening the cross section of the tubular film so as to ease insertion of medical apparatus components,
wherein the auxiliary means, which may in an unfolded state form a tubular sleeve (2), is inserted close-fittingly and concentrically into the first end (14) of the tubular film (1) and projects outwards beyond this first end (14), and wherein the sleeve (2) consists of more rigid material than the tubular film (1), characterised in that the sleeve (2) is provided in the axial direction thereof with at least four longitudinally extending predetermined fold lines (21, 22, 23, 24), of which the first two diametrically opposed predetermined fold lines (21, 24) enable the sleeve (2) to be folded flat and the other predetermined fold lines (22, 23) lying between these first two predetermined fold lines (21, 24) enable tubular opening-out of the sleeve (2) and thus of the first end of the tubular film (1) by pressure on the first predetermined fold lines (21, 24).

2. A tubular film covering according to claim 1,
characterised in that the sleeve (2) comprises two lugs (25, 26) projecting out of the tubular film (1), which lugs (25, 26) are connected with the main portion of the sleeve (2) via transverse fold lines (29, 30) and may be folded outwards in a direction perpendicular to the longitudinal axis of the sleeve (2).

3. A tubular film covering according to claim 1 or claim 2, characterised in that the sleeve (2) comprises at least one further lug (27, 28) projecting out of the tubular film, which lug (27, 28) comprises widening slits (32, 33) for accommodating cables, tubing and the like.

4. A tubular film covering according to any one of the preceding claims,
characterised in that the sleeve (2) is made of cardboard material.

5. A tubular film covering according to any one of the preceding claims,
characterised in that the first end (14) of the tubular film (1) is connected to the tubular sleeve (2) by manually releasable fixing means (15).

6. A tubular film covering according to claim 5,
characterised in that the fixing means take the form of a breakable weld joint (15).

7. A tubular film covering according to any one of the preceding claims,
characterised in that the predetermined fold line(s) (22, 23) lying between the first two predetermined fold lines (21, 24) are at different distances from each of the adjacent predetermined fold lines in the circumferential direction of the tubular sleeve (2), in such a way that, when the cross section of the tubular sleeve (2) or the tubular film (1) is opened out, an elongate, rectangular cross section is obtained.

## Revendications

1. Recouvrement en forme de film tubulaire pour appareils médicaux, comprenant un film tubulaire ou housse de forme allongée, qui est disposé à plat pour le stockage et le transport et dont la longueur est raccourcie par un pliage à emmanchement de type télescopique et qui peut être étendu lors de son utilisation à toute sa longueur, et qui présente une première et une seconde extrémité dont au moins la première extrémité présente des dispositifs auxiliaires pour l'ouverture de la section droite de la housse afin de faciliter l'introduction de parties de l'appareil médical, les dispositifs auxiliaires pouvant former, à l'état ouvert par dépliage, un manchon tubulaire (2) introduit concentriquement dans la première extrémité (14) de la housse (1), en s'appliquant intimement contre celle-ci et en dépassant à l'extérieur au-delà de la première extrémité (14) de la housse, et le manchon (2) étant formé d'un matériau plus raide que la housse (1), caractérisé en ce que le manchon (2) est pourvu d'au moins quatre lignes (21, 22, 23, 24) prévues pour former des plis, lesquelles s'étendent longitudinalement en direction axiale du manchon, dont deux premières lignes de pliage (21, 22), situées diamétralement l'une en face de l'autre, permettent le pliage à plat du manchon (2) et dont les autres lignes de pliage (22, 23) situées entre ces deux premières lignes de pliage (21, 24), permettent l'ouverture par dépliage du manchon tubulaire (2) et par suite de la première extrémité de la housse (1) sous l'effet d'une pression exercée sur les premières lignes de pliage (21, 24).

2. Recouvrement en forme de film tubulaire selon la revendication 1, caractérisé en ce que le manchon (2) comporte deux rabats (25, 26) qui dépassent de la housse (1), sont reliés par des lignes transversales de pliage (29, 30) à la partie principale du manchon (2) et sont dépliables vers l'extérieur en direction perpendiculaire à l'axe longitudinal du manchon (2).

3. Recouvrement en forme de film tubulaire selon la revendication 1 ou 2, caractérisé en ce que le manchon (2) présente au moins un autre rabat (27, 28) dépassant de la housse et qui présente des fentes (32, 33) avec des élargissements pour la réception de câbles, tuyaux souples et analogues.

4. Recouvrement en forme de film tubulaire selon une des revendications précédentes, caractérisé en ce que le manchon (2) est en carton.

5. Recouvrement en forme de film tubulaire selon une des revendications précédentes, caractérisé en ce que la première extrémité (14) de la housse (1) est reliée au manchon tubulaire (2) par des moyens de fixation (15) détachables manuellement.

6. Recouvrement en forme de film tubulaire selon la revendication 5, caractérisé en ce que les moyens de fixation sont formés par une liaison soudée (15) détachable.

7. Recouvrement en forme de film tubulaire selon une des revendications précédentes, caractérisé en ce que la ou les ligne(s) de pliage (22, 23) se trouvant entre les deux premières lignes de pliage (21, 24) dans la direction circonférentielle du manchon tubulaire (2), présente(nt) des distances différentes des lignes de pliage voisines, de manière que, lors de l'ouverture du manchon tubulaire (2) et de la housse (1), on obtienne une section droite ouverte de forme rectangulaire allongée.
